(19) Europäisches Patentamt
European Patent Office
Office européen des brevets

(11) EP 4 115 967 A1

(12) EUROPEAN PATENT APPLICATION
published in accordance with Art. 153(4) EPC

(43) Date of publication:
11.01.2023 Bulletin 2023/02

(51) International Patent Classification (IPC):
B01D 39/14 (2006.01)    A61M 1/02 (2006.01)
A61M 1/34 (2006.01)

(21) Application number: 21764736.1

(22) Date of filing: 02.03.2021

(52) Cooperative Patent Classification (CPC):
A61M 1/02; A61M 1/34; B01D 39/14

(86) International application number:
PCT/JP2021/007838

(87) International publication number:
WO 2021/177272 (10.09.2021 Gazette 2021/36)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 04.03.2020 JP 2020036663

(71) Applicant: Asahi Kasei Medical Co., Ltd.
Tokyo 100-0006 (JP)

(72) Inventors:
• YOSHIDA, Akiko
Tokyo 100-0006 (JP)
• NAKAMURA, Kazuhiko
Tokyo 100-0006 (JP)
• YOKOMIZO, Tomohisa
Tokyo 100-0006 (JP)

(74) Representative: dompatent von Kreisler Selting
Werner -
Partnerschaft von Patent- und Rechtsanwälten
mbB
Deichmannhaus am Dom
Bahnhofsvorplatz 1
50667 Köln (DE)

(54) BLOOD TREATMENT FILTER

(57) The present invention has an object to provide a blood processing filter that is excellent in the filtration rate. The object can be solved by a blood processing filter comprising a container having an inlet and an outlet for blood, and a filter medium disposed between the inlet and the outlet of the container, wherein the filter medium comprises a filter element, an average thickness of the filter medium is 7 to 12 mm, and a standard deviation in thickness of the filter medium is 0.30 to 0.80 mm.

[Figure 2]

**Description**

Technical Field

The present invention relates to blood processing filters.

Background Art

Whole blood collected from donors is used as raw materials for blood component preparations such as red blood cell preparations, platelet preparations, and plasma preparations, however whole blood contains unpreferable components such as microaggregates and white blood cells that cause various blood transfusion adverse side effects. For this reason, unpreferable components are usually removed after blood collection or before a blood component preparation is used.

As methods for removing unpreferable components such as white blood cells from whole blood or blood component preparations, filter methods in which blood processing filters comprising a filter medium consisting of a fiber assembly such as a non-woven fabric or a pore structure having continuous pores have been widely used due to an easy operation and a low cost.

It is considered that the mechanism of white blood cell removal by the filter method is achieved when the white blood cells that primarily contact the filter medium surface are adhered to or adsorbed onto the filter medium surface. Thus, a contact frequency of the filter medium and white blood cells is increased to enhance a white blood cell removal ability, specifically, a surface area per filter medium unit volume is increased by decreasing a fiber diameter of a fiber or a pore diameter of a pore structure or by increasing a bulk density of these that structure the filter medium thereby to enhance the white blood cell removal ability (Patent Literature 1). These methods manage to enhance the white blood cell removal ability, however, along therewith, an increased pressure loss caused by the filter medium when processing blood reduces a flow rate and extends filtration time.

Studies have been investigated, as a way of mitigating the pressure loss, on imparting surface modifications or surface treatments with the filtration medium to improve blood wettability of the filter medium (Patent Literature 2). These methods can inhibit the filtration time extension by the pressure loss mitigation, however, it is difficult to maintain a good white blood cell removal ability.

In the case of removing unpreferable components from refrigerated blood (hereinafter, referred to as "refrigerated blood"), the refrigerated blood has an increased viscosity thereby slowing down a filtration flow rate whereby white blood cells tend to likely adsorb onto the filtration medium. Further, aggregates caused in the refrigerated blood while stored block flow passages of the filter medium thereby resulting in a low filtration rate.

Citation List

Patent Literature

  Patent Literature 1: Japanese Patent Laid-Open No. H2-203909
  Patent Literature 2: Japanese Patent Laid-Open No. 2007-50013

Summary of Invention

Technical Problem

In view of the above-mentioned problems, the present invention has an object to provide a blood processing filter that is excellent in the filtration rate.

Solution to Problem

The present inventors have conducted extensive studies and have consequently found that the above problems can be solved when a filter medium having a variation in thickness is used.

The present invention comprises the following embodiments.

  [1] A blood processing filter comprising:

a container having an inlet and an outlet for blood, and
a filter medium disposed between the inlet and the outlet of the container,
wherein the filter medium comprises a filter element,
an average thickness of the filter medium is 7 to 12 mm, and
a standard deviation in thickness of the filter medium is 0.30 to 0.80 mm.

[1-1A] The blood processing filter according to [1], wherein a standard deviation in thickness of the filter element is 0.002 to 0.015 mm.

[1-1B] The blood processing filter according to [1] or [1-1A], wherein a standard deviation in thickness of the filter element is 0.002 to 0.010 mm.

[1-2A] The blood processing filter according to any of [1] to [1-1B], wherein a basis weight of the filter element is 45 to 150 g/m$^2$.

[1-2B] The blood processing filter according to any of [1] to [1-2A], wherein a basis weight of the filter element is 53 to 150 g/m$^2$.

[1-2C] The blood processing filter according to any of [1] to [1-2B], wherein a basis weight of the filter element is 68 to 150 g/m$^2$.

[1-2D] The blood processing filter according to any of [1] to [1-2C], wherein a basis weight of the filter element is 85 to 95 g/m$^2$.

[2] The blood processing filter according to any of [1] to [1-2D], wherein the standard deviation in thickness of the filter medium is 0.40 to 0.70 mm.

[3] The blood processing filter according to any of [1] to [2], wherein the container consists of an inlet-side container material having the inlet and an outlet-side container material having the outlet,

wherein the inlet-side container material and the outlet-side container material are welded at the periphery thereof while pinching the filter medium, and
a thickness of the welded part is 1.2 mm to 1.8 mm.

[3-1] The blood processing filter according to any of [1] to [3], wherein the thickness of the welded part is 1.3 to 1.6 mm.

[4] The blood processing filter according to any of [1] to [3-1], wherein the average thickness of the filter medium is 8 to 11 mm.

[4-1] The blood processing filter according to any of [1] to [4], wherein the average thickness of the filter medium is 9 to 10 mm.

[5] The blood processing filter according to any of [1] to [4-1], wherein a bulk density of the filter element is 0.14 to 0.30 g/cm$^3$.

[6] The blood processing filter according to any of [1] to [5], wherein a bulk density of the filter element is 0.18 to 0.30 g/cm$^3$.

[7] The blood processing filter according to any of [1] to [6], wherein an average thickness of the filter element is 0.38 to 0.50 mm.

[7-1] The blood processing filter according to any of [1] to [7], wherein an average thickness of the filter elements is 0.40 to 0.45 mm.

[8] The blood processing filter according to any of [1] to [7-1], wherein an effective filtration area of the filter medium is 40 to 50 cm$^2$.

[8-1] The blood processing filter according to any of [1] to [8], wherein an effective filtration area of the filter medium is 40 to 45 cm$^2$.

Advantageous Effects of the Invention

According to the present invention, a blood processing filter that is excellent in the filtration rate can be provided.

Brief Description of Drawing

[Figure 1] Figure 1 is a diagrammatic drawing of a blood processing filter, which is an embodiment of the present invention.
[Figure 2] Figure 1 is a cross section drawing of the diagrammatic drawing of Figure 1.
[Figure 3] Figure 3 is a cross section drawing (cross section in the thickness direction) of a blood processing filter, which is an embodiment of the present invention. The figure shows, in a container, a plurality of filter elements present in a slack state causing non-homogeneous voids between the filter elements.
[Figure 4] Figure 4 shows the parts to be used for measuring an average thickness of the filter medium and a standard deviation in thickness.
[Figure 5] Figure 5 is a schematic drawing of the experimental device used for the tests in Examples.

Description of Embodiments

Hereinafter, embodiments of the present invention will be described in detail. The present invention is not limited to the following embodiments and can be carried out in various modifications within the spirit and scope thereof.
Hereinafter, unless otherwise specified, the term "blood" includes blood and blood component-containing liquids. Examples of the blood component-containing liquid include blood preparations. Examples of the blood preparation include whole blood preparations, red blood cell preparations, platelet preparations, and plasma preparations.

<Blood processing filter>

An embodiment of the present invention relates to a blood processing filter comprising a container having an inlet and an outlet for blood, and a filter medium disposed between the inlet and the outlet of the container, wherein the filter medium comprises a filter element, an average thickness of the filter medium is 7 to 12 mm, and a standard deviation in thickness of the filter medium is 0.30 to 0.80 mm.
The blood processing filter of the present embodiment comprises a filter medium having a variation in thickness, and the variation is expressed by a standard deviation in thickness. The use of a filter medium having a variation in thickness can achieve an excellent filtration rate. The reason for achieving such an effect is, for example, presumed to be the securement of flow passages through which blood flows due to a variation in thickness, but the present invention is not limited to such a presumed mechanism.
In the blood processing filter of the present embodiment, a filter medium is disposed so that blood that enters the inlet of the container passes through the filter medium before exiting from the outlet.

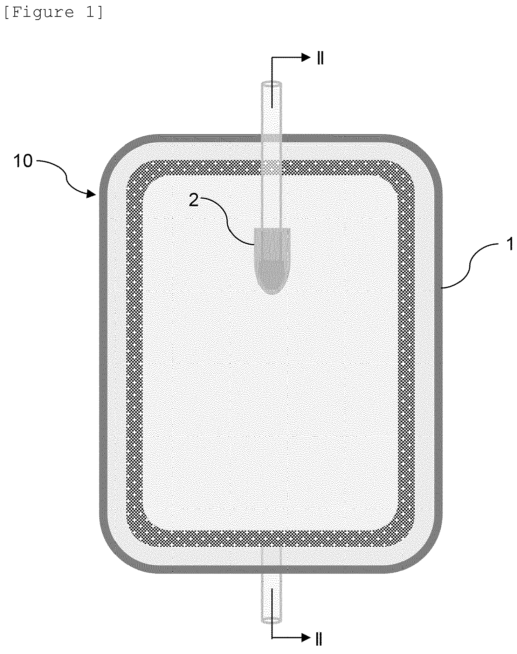

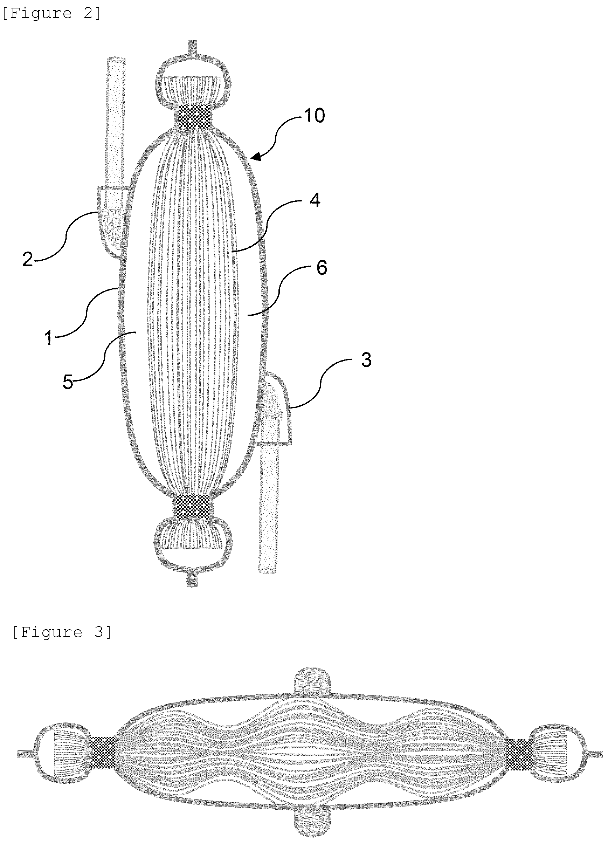

Figure 1 is a diagrammatic drawing showing an example of the blood processing filter of the present embodiment, Figure 2 is a cross section drawing of Figure 1 taken along the line II-II. As shown in Figure 1 and Figure 2, a blood processing filter 10 comprises a flat container 1 and a filter medium 4 held inside thereof. The container 1 holding the filter medium 4 is structured by an inlet-side container material having an inlet 2 and an outlet-side container material having an outlet 3. The space in the flat container 1 is partitioned by the filter medium 4 into a space at the inlet side 5 and a space at the outlet side 6. In the blood processing filter 10, the inlet-side container material and the outlet-side container material are disposed in such a way as to pinch the filter medium 4, and the two container materials grip the outer edge (periphery) of the filter medium 4 with a gripper each formed at a part thereof. The gripper can be a welded part or the like.

[Container]

The container for the blood processing filter of the present embodiment has an inlet for unprocessed blood and an outlet for processed blood. The container is not particularly limited, and a container used for general blood processing filters can be employed.
The shape of the container is not particularly limited and can be a shape according to the shape of the filter medium. For example, when the filter medium is plate-like, the container can be a polygon (for example, tetragons and hexagons), a round shape, an oval shape, and the like to meet the shape of the filter medium. When the filter medium is cylindrical, the container can also be cylindrical.
The material of the container is not particularly limited and can employ a material used for containers of general blood processing filters. Example of the material of the container include flexible resins and hard resins.
Examples of the flexible resin include soft polyvinyl chloride, polyurethane, an ethylene-vinyl acetate copolymer, polyolefins (for example, polyethylene and polypropylene), a hydrogenated product of a styrene-butadiene-styrene copolymer, and a styrene-isoprene-styrene copolymer or a hydrogenated product thereof.
Examples of the hard resin include a phenol resin, an acrylic resin, an epoxy resin, a formaldehyde resin, a urea resin, a silicon resin, an ABS resin, nylon, polyurethane, polycarbonate, vinyl chloride, polyethylene, polypropylene, polyester, and a styrene-butadiene copolymer.

[Filter medium]

The average thickness of the filter medium for the blood processing filter of the present embodiment is 7 to 12 mm, preferably 8 to 11 mm, and more preferably 9 to 10 mm. When an average thickness of the filter medium is within the above range, the white blood cell removal performance can be enhanced. The average thickness of the filter medium can be suitably adjusted by, for example, changing the number and thickness of the filter elements structuring the filter medium. The average thickness of the filter medium can be measured by the method described in Examples.

The standard deviation in thickness of the filter medium for the blood processing filter of the present embodiment is 0.30 to 0.80 mm, and preferably 0.40 to 0.70 mm. When a standard deviation is within the above range, the balance with a blood loss volume becomes better and the filtration rate can also be enhanced. The present invention is presumed to enhance the filtration rate when a standard deviation is 0.30 mm or more because a variation in thickness of the filter medium is greater thereby forming new flow passages through which blood flows, however the present invention is not limited to this presumed mechanism. When a standard deviation is 0.80 mm or less, a decrease in filtration efficiency caused by flow passages that are increased too much is presumably prevented. The standard deviation in thickness of the filter medium can be measured by the method described in Examples.

For preparing a white blood cell-removed plasma preparation, whole blood stored preferably at room temperature or refrigerated is centrifuged within 72 hours, further preferably within 48 hours, particularly preferably within 24 hours, and most preferably within 12 hours, after blood collection. Preferably, white blood cells are removed from a plasma preparation stored at room temperature or refrigerated using a blood processing filter F at room temperature or under refrigeration within 120 hours, further preferably within 72 hours, particularly preferably within 24 hours, and most preferably within 12 hours, after blood collection thereby to obtain a white blood cell-removed plasma preparation. In the case of white blood cell removal after storage, white blood cells are removed using the blood processing filter F from a plasma preparation preferably stored at room temperature, refrigerated, or frozen within 24 hours before use thereby to obtain a white blood cell-removed plasma preparation. Particularly, in Europe and Japan, a white blood cell-removed plasma preparation before storage is not allowed to use as FFP (fresh frozen plasma) unless prepared within 24 hours or within 8 hours after blood collection, respectively. When filtration time is extended and the specified time is exceeded, such a preparation must be used for a different purpose such as raw material plasma thereby notably reducing work efficiency. Thus, appropriate completion in the filtration time is also important for improved work efficiency.

Further, the securement of reasonable flow passages demonstrates an effect to enhance the blood recovery yield without remaining blood inside the blood processing filter.

The number of white blood cell filtrations currently practiced in the blood transfusion market is allegedly 50 million times a year. Given the case where the new filter succeeds in reducing a whole blood loss volume of 0.1 mL against a filter for whole blood having a blood loss volume of 35 mL in the filter, an annual whole blood loss reduction volume in the case of practicing 1/10 of the number of blood collections a year, that is 5 million times, is as follows.

$$1/10000 \ (L) \ \times \ 500 \ \times \ 10000 \ = \ 500 \ L$$

It is obvious that a blood loss reduction even as small as 0.1 mL still causes an enormous loss in blood recovery yield in the markets of developed countries (for example, Japan and Europe) where the number of annual blood collections is high and preparation systems for blood preparations have been established.

The standard deviation in thickness of the filter medium can be suitably adjusted by, for example, changing the basis weight of filter element. More specifically, when a filter medium in which only high basis weight filter elements are laminated is pinched between the inlet-side container material and the outlet-side container material, and the filter medium and the two container materials are welded at the outer edge thereof, a standard deviation in thickness of the filter medium tends to be large. Conversely, when low basis weight filter elements are used at least partly, a standard deviation in thickness of the filter medium tends to be small. The reason for these differences is presumed that high basis weight filter elements, which are more stretched than low basis weight filter elements, form slack when welded to the container materials, and this slack causes a variation in thickness of the filter medium. In the case of using low basis weight filter elements, processed filter elements are laminated in addition to pleating and embossing for imparting stretchiness with the filter medium, the filter medium is pinched between the inlet-side container material and the outlet-side container material, and the filter medium and the two container materials are welded at the outer edge thereof whereby the standard deviation in thickness of the filter medium tends to be large.

The standard deviation in thickness of the filter medium can also be adjusted without changing the basis weight of filter element. Typically, the container consists of the inlet-side container material having the inlet and the outlet-side container material having the outlet, wherein the inlet-side container material and the outlet-side container material are welded at the periphery thereof while pinching the filter medium. Changing welding conditions herein can adjust the slack and thereby can adjust the standard deviation in thickness of the filter medium. In the case of using a high-frequency welding machine, the magnitude of the slack can be controlled by adjusting welding current and welding time when oscillating a high frequency. Welding conditions can be suitably adjusted according to an intended standard deviation. The indicator for determining the welding intensity includes, for example, a thickness at a welded part. The thicker a thickness at a welded part is, the weaker the welding tends to be, whereas the thinner a thickness at a welded part is, the stronger the welding tends to be. Typically, when a thickness at a welded part is 1.2 to 1.8 mm, the filter function is sufficiently met, and the thickness is more preferably 1.3 to 1.6 mm.

Alternatively, the standard deviation in thickness of the filter medium can also be adjusted by a method in which the filter medium is fit into a rather small formwork and welded in a slack state, a method of inserting a spacer between filter elements and the like.

It is preferable that the container be free from slack when adjusting the standard deviation in thickness of the filter medium. That is, it is preferable that the blood processing filter be not curved as a whole.

It is preferable that, as an example, the container consist of an inlet-side container material having the inlet and an outlet-side container material having the outlet, wherein the inlet-side container material and the outlet-side container material are welded at the periphery thereof while pinching the filter medium, and the blood processing filter is present at all positions on a line connecting two welded parts intersecting with any direction (hereinafter, referred to as the "first direction") that orthogonally intersects with the thickness direction of the blood processing filter, and the blood processing filter is present at all positions on a line connecting two welded parts intersecting with a second direction that orthogonally intersects with the first direction.

For example, Figure 2 is a cross section of the blood processing filter cut in the longitudinal direction, and Figure 3 is a cross section of the blood processing filter cut in the short direction. When the longitudinal direction is the first direction, the short direction orthogonally intersecting therewith is the second direction. In Figure 2, the blood processing filter is present at all positions on a line connecting the upper welded part and the lower welded part intersecting with the longitudinal direction (first direction), and in Figure 3, the blood processing filter is present at all positions on a line connecting the left welded part and the right welded part intersecting with the short direction (second direction), for which the blood processing filter is not curved as a whole.

The effective filtration area of the filter medium for the blood processing filter of the present embodiment is 40 to 50 cm$^2$, and preferably 40 to 45 cm$^2$. The effective filtration area refers to the area that is actually used for the filtration. When an effective filtration area of the filter medium is within the above range, the filtration rate, white blood cell removal performance, and blood recovery rate can be enhanced. The effective filtration area of the filter medium can be suitably adjusted by, for example, changing the number, specific surface area, and mass per unit area of filter elements structuring the filter medium. The effective filtration area of the filter medium can be measured by the method described in Examples.

[Filter element]

The filter medium preferably has a structure in which a plurality of filter elements are laminated. The number of the filter elements to be laminated is not limited and can be suitably determined according to an intended thickness of the filter medium. When the filter medium comprises a plurality of filter elements, each of the filter elements can be the same or different.

The average thickness of the filter elements is preferably 0.38 to 0.50 mm, and more preferably 0.40 to 0.45 mm. When an average thickness of the filter elements is within the above range, the blood recovery rate can be enhanced. The average thickness of the filter elements can be measured by the method described in Examples.

The thickness of the filter elements preferably has a low variation. For example, the standard deviation in thickness of the filter elements is preferably 0.002 to 0.015 mm, and more preferably 0.002 to 0.010 mm. The standard deviation in thickness of the filter elements can be measured by the method described in Examples.

The filter medium, despite being structured by laminating filter elements with a low variation in thickness, has a greater variation in thickness thereof, and examples of the reason for this include the presence of non-homogeneous voids between the filter elements. That is, the filter medium is thicker at a position where a large void is present, whereas the filter medium is thinner at a position where a void is small. This causes variations in thickness of the filter medium. Voids can function as flow passages for blood thereby contributing to the enhancement of the filtration rate.

The bulk density of the filter element is preferably 0.14 to 0.30 g/cm$^3$, and more preferably 0.18 to 0.30 g/cm$^3$. When a bulk density of the filter element is within the above range, the white blood cell removal performance can be enhanced. The bulk density of the filter element can be measured by the method described in Examples.

The form of filter element is not particularly limited as long as it is suitable for blood processing. Example of the form of filter element include woven fabric, non-woven fabric, and porous membrane.

The material for filter element is not particularly limited as long as it does not detrimentally affect blood. Examples of the material for filter element include polyester, polyolefin, polyacrylonitrile, polyamide, polystyrene, polymethyl methacrylate, polyvinyl fluoride, polyurethane, polyvinyl alcohol, polyvinyl acetal, polysulfone, polyvinylidene fluoride, polytrifluorochlorovinyl, a vinylidene fluoride-tetrafluoroethylene copolymer, polyether sulfone, polyacrylate, a butadiene-acrylonitrile copolymer, a polyether-polyamide block copolymer, an ethylene-vinyl alcohol copolymer, cellulose, and cellulose acetate.

When the filter element is a non-woven fabric, the thickness and bulk density of the non-woven fabric can be arbitrarily adjusted by adjusting conditions when producing the non-woven fabric. Examples of the production method of a non-woven fabric which is easy to adject fiber structure include a melt blown method, and a non-woven fabric having a predetermined thickness and bulk density can be obtained by investigating spinning factors such as the resin viscosity, melting temperature, discharge quantity per pore, heated gas temperature, heated gas pressure, distance between a spinning spout and an accumulation net. For the production method of a filter material (non-woven fabric) having a reasonable thickness and bulk density, reasonable production conditions can be determined by considering selections of the above technical ideas and production process based on the publicly known information (for example, Non Patent Literature: "Fushokufu no Kiso to Oyo" ("Foundation and Application of non-woven fabric" in English), P.119-127, published on August 25, 1993, Japan Textile Machinery Association).

In the present embodiment, for example, polybutylene terephthalate (PBT) non-woven fabrics having different bulk densities and thicknesses can be obtained by adjusting the conditions below in the melt blown method. Examples of the spinning conditions in the melt blown method include the number of spinning spouts of a melt blown die, pore discharge quantity, and heated air volume, and these can be arbitrarily set.

The number of spinning spouts of a melt blown die can be typically set to be 5 holes/cm or more and 30 holes/cm or less.

The pore discharge quantity can be typically set to be 0.12 g/ (min·hole) or more and 0.20 g/ (min·hole) or less.

The heated air volume can be typically set to be 100 $Nm^3$/hr or more and 400 $Nm^3$/hr or less.

The basis weight of the filter element is not particularly limited and preferably 45 to 150 $g/m^2$, more preferably 53 to 150 $g/m^2$, further preferably 68 to 150 $g/m^2$, and particularly preferably 85 to 95 $g/m^2$. It is preferable that the basis weight of all filter elements comprised in the filter medium be within the above range. When a basis weight of the filter element is within the above range, the white blood cell removal performance can be enhanced.

[Purpose of use]

The blood processing filter of the present embodiment can be used for processing blood. The "processing" means the removal of unpreferable components (for example, white blood cells) from blood. The blood processing filter of the present embodiment is particularly suitable for processing refrigerated blood but not particularly limited thereto. As described above, when refrigerated blood is filtered, a filtration rate tends to slow down, however the use of the blood processing filter of the present embodiment enables quick processing of refrigerated blood.

<Production method of blood processing filter>

The production method of the blood processing filter can be carried out so that variations in thickness of the filter medium are caused and is not particularly limited. Examples of the method for causing variations in thickness of the filter medium include pinching a filter medium in which high basis weight filter elements are laminated between the inlet-side container material and the outlet-side container material and welding the filter medium and the two container materials at the outer edge thereof. High basis weight filter elements are likely to be a slack shape in the formed container, whereby voids are caused non-homogeneously between the filter elements thereby causing variations in thickness of the filter medium (for example, see Figure 3).

Examples of different methods for causing variations in thickness of the filter medium include the method described in the above section [Filter medium].

Examples

Hereinafter, the present invention will be described in further details in reference to examples and comparative examples, but the technical scope of the present invention is not limited thereto.

<Measurement method>

[Average thickness of filter medium]

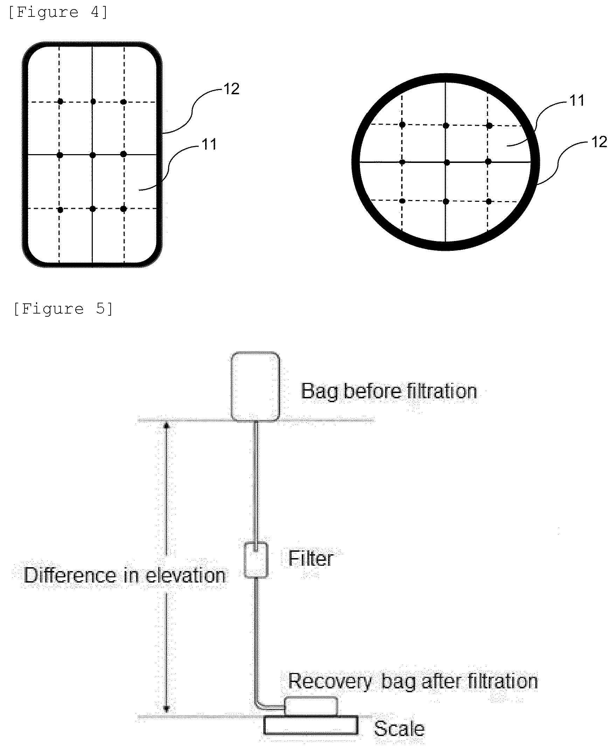

Housings inside the joint parts (welded parts) of the blood processing filter (product) are removed to expose the filter medium. As shown in Figure 4, two center lines (solid lines) intersecting at the center of a filter medium 11 are drawn, four lines (dotted lines) equally dividing the areas, which is from each of the center lines to the inside of the welded part 12, are drawn in parallel on both sides of the center lines, and the thicknesses at 9 points at which these lines intersect are measured using a thickness gauge (Model: SM-114, maker: TECLOCK) with a measuring force of 3.2 $N/cm^2$ to define the average thereof as the average thickness of the filter medium. (The measuring force is decided to be 2.5 $N/0.785 cm^2$ = 3.2 $N/cm^2$ because the user's manual of the thickness gauge includes a description of 2.5 N being applied to an object to be measured and the area (a circle having a 1 cm-diameter) at which the thickness gauge contacts the non-woven fabric is 0.785 cm$^2$.)

[Standard deviation in thickness of filter medium]

The standard deviation was determined based on the thicknesses at 9 points measured in the above section [Average thickness of filter medium]. The present measurement method can precisely measure the standard deviation in thickness of the filter medium to at least 2 decimal places.

[Average thickness of filter elements]

Eleven sample sheets each having a size of 5 cm $\times$ 20 cm were cut out from filter elements, and 1 point per sheet was measured using a thickness gauge (Model: ID-C112, maker: Mitutoyo) to define the average thereof as the average thickness of the filter elements.

[Standard deviation in thickness of filter elements]

The standard deviation was determined based on the thicknesses at the 11 points measured in the above section [Average thickness of filter elements].

[Bulk density of filter element]

$$\text{Bulk density (g/cm}^3) \text{ of filter element = basis}$$
$$\text{weight of filter element (g/m}^2)/\text{average thickness (mm) of}$$
$$\text{filter elements}/1000$$

[Basis weight of filter element]

A sample having a size of 5 cm $\times$ 20 cm was cut out from a filter element and put on a scale (Model: XP205, maker: METTLER TOLEDO) to measure a mass thereof. A basis weight of the filter element was calculated based on the obtained value in accordance with the following equation.

$$\text{Basis weight = mass of a filter element (g)} \div \text{area}$$
$$\text{of the filter element (m}^2)$$

[Average thickness at welded pars]

The center part of each side at a welded part was measured using a micrometer (maker: Mitutoyo, Model: 331-261), or 4 points at equally spaced intervals were measured in the case of a round shape, to define the average thereof as the thickness at the flange welded part.

[Effective filtration area of filter medium]

The filter medium and flexible sheets were welded at the peripheral part to define a filter medium area inside the welded part as the effective filtration area.

<Test method>

[Filtration time]

In the test on "residual white blood cell count" described below, the time (minute) from the start of flowing a red blood cell preparation through a blood processing filter, to the stop of a mass increase of a recovery bag for the filtered red blood cell preparation was defined as the filtration time (minute). The stop of a mass increase of the recovery bag refers to the point in time at which a mass change of the recovery bag was 0.1 g or less when a mass of the recovery bag was measured by the minute from the start of filtration. In the present examples, the filtration time was calculated by including the last 1 minute at which the stop of a mass increase was decided.

Evaluation criteria were as follows.

[Evaluation criteria]

A: Filtration time is less than 25 minutes, and reduction in filtration time is excellent.
B: Filtration time is more than 25 minutes and 30 minutes or less, and reduction in filtration time is good.
C: Filtration time is more than 30 minutes, and reduction in filtration time is not excellent.

[Residual white blood cell count]

A red blood cell preparation prepared in accordance with the European Standard (the Guide to the Preparation, Use and Quality Assurance of Blood Components, 19th edition, (2017)) was used as a blood preparation, filtered and recovered using the blood processing filters of Examples and Comparative Examples with a natural difference in elevation of 110 cm thereby to obtain a blood preparation after filtration. The difference in elevation herein, as shown in Figure 5, was defined as the distance from the lowest part of the bag containing the red blood cell preparation before filtration to the lowest part of the recovery bag for the red blood cell preparation after filtration (in the example of Figure 5, to the top plate of the scale).

Subsequently, a residual white blood cell count after filtration was calculated in accordance with the following equation.

$$\text{Residual white blood cell count after filtration} = \log (\text{white blood cell concentration in blood preparation after filtration} \times \text{amount of blood preparation after filtration})$$

The measurement of a white blood cell concentration in the blood preparation after and before filtration was carried out using a white blood cell count measuring kit "LeucoCOUNT" manufactured by Becton, Dickinson and Company (BD) and a flow cytometer FACS Canto II manufactured by BD.

Evaluation criteria were as follows.

[Evaluation criteria]

A: Residual white blood cell count is less than 5.0, and white blood cell removal ability is excellent.
B: Residual white blood cell count is 5.0 or more and less than 5.5, and white blood cell removal ability is good.
C: Residual white blood cell count is 5.5 or more, and white blood cell removal ability is low.

[Blood loss volume]

In the test on [Residual white blood cell count] described above, a weight (g) of the blood processing filter after completion of filtration was measured, and a filter weight (g) before filtration was subtracted therefrom to calculate a blood loss volume.

[Evaluation criteria]

A: Blood loss volume is less than 28 ml, and blood recovery rate is excellent.
B: Blood loss volume is 28 ml or more and less than 30 ml, and blood recovery rate is good.
C: Blood loss volume is 30 ml or more, and blood recovery rate is low.

<Production of blood processing filter>

[Example 1]

Twenty-three non-woven fabrics having a filter element thickness of 0.42 (mm), a filter element bulk density of 0.21 (g/cm$^3$), and a filter element basis weight of 88.2 (g/m$^2$) were laminated and cut to a size of 91 mm × 74 mm using a razer cutter to make a filter medium.
This filter medium was pinched between two flexible polyvinyl chloride resin sheets having a port to be an inlet or an outlet for blood, and the filter medium and the peripheral part of the flexible sheets were integrated by welding using a high-frequency welding machine. The inner side of the welded part had a longitudinal dimension of 74 mm and a horizontal dimension of 57 mm, and was the effective filtering part in rectangular with curved corners with an effective filtration area of 42 cm$^2$. In the effective filtering part, the filter medium had a thickness of 8.00 mm and a standard deviation of 0.30 mm.
The peripheral part of the flexible sheets was further integrated by welding to make a blood processing filter having a thickness at the welded part of 1.45 mm. High-pressure steam sterilization was carried out on the blood processing filter at 115°C for 59 minutes, and then each of the tests described above was carried out.

[Example 2]

A blood processing filter was produced in the same manner as in Example 1 except that the number of filter elements to be laminated was increased by two and the welding conditions for the filter medium and the peripheral part of the flexible sheets were changed. The filter medium had a thickness of 9.60 mm, a standard deviation of 0.43 mm, and a thickness at the welded part of 1.45 mm.

[Example 3]

A blood processing filter was produced in the same manner as in Example 1 except that the number of filter elements to be laminated was increased by four and the welding conditions for the filter medium and the peripheral part of the flexible sheets were changed. The filter medium had a thickness of 11.00 mm, a standard deviation of 0.80 mm, and a thickness at the welded part of 1.30 mm.

[Example 4]

A filter medium was made in the same manner as in Example 2 except that the filter element had a bulk density of 0.16 (g/cm$^3$) and a basis weight of 67.2 (g/m$^2$). The filter medium was put in a 91 mm × 73.9 mm formwork when welding the filter medium and the peripheral part of the flexible sheets using a high-frequency welding machine, and the filter medium and the peripheral part of the flexible sheets were integrated by welding. The filter medium had a thickness of 9.60 mm, a standard deviation of 0.43 mm, and a thickness at the welded part of 1.45 mm.

[Example 5]

A filter medium was made in the same manner as in Example 2 except that the filter element had a bulk density of 0.12 (g/cm$^3$) and a basis weight of 50.4 (g/m$^2$). The razer-cut filter medium was put in a 91 mm × 73.8 mm formwork when welding the filter medium and the peripheral part of the flexible sheets using a high-frequency welding machine to integrate the filter medium and the peripheral part of the flexible sheets by welding. The filter medium had a thickness of 9.60 mm, a standard deviation of 0.43 mm, and a thickness at the welded part of 1.45 mm.

[Example 6]

A blood processing filter was produced in the same manner as in Example 2 except that the filter element having a thickness of 0.35 (mm) and a basis weight of 73.5 (g/m$^2$) was used and the number of filter elements to be laminated was increased by three. The filter medium had a thickness of 9.60 mm, a standard deviation of 0.43 mm, and a thickness at the welded part of 1.45 mm.

[Example 7]

A blood processing filter was produced in the same manner as in Example 2 except that the filter element having a thickness of 0.55 (mm) and a basis weight of 115.5 (g/m$^2$) was used and the number of filter elements to be laminated was decreased by three. The filter medium had a thickness of 9.60 mm, a standard deviation of 0.43 mm, and a thickness at the welded part of 1.45 mm.

[Example 8]

A blood processing filter was produced in the same manner as in Example 2 except that an effective filtration area was 35 cm$^2$. The filter medium had a thickness of 9.60 mm, a standard deviation of 0.43 mm, and a thickness at the welded part of 1.45 mm.

[Example 9]

A blood processing filter was produced in the same manner as in Example 2 except that an effective filtration area was 55 cm$^2$. The filter medium had a thickness of 9.60 mm, a standard deviation of 0.43 mm, and a thickness at the welded part of 1.45 mm.

[Example 10]

A blood processing filter was produced in the same manner as in Example 2 except that a pleated filter element having a bulk density of 0.18 (g/m$^3$), a thickness of 0.25 (mm), and a basis weight of 45 (g/m$^2$) was used and the number of filter elements to be laminated was increased by ten. The filter medium had a thickness of 8.00 mm, a standard deviation of 0.30 mm, and a thickness at the welded part of 1.50 mm.

[Example 11]

A filter medium was produced in the same manner as in Example 1. The filter medium and the peripheral part of the flexible sheets were integrated by welding in the same manner as in Example 5. The filter medium had a thickness of 8.00 mm, a standard deviation of 0.35 mm, and a thickness at the welded part was 1.45 mm.

[Example 12]

A filter medium was produced in the same manner as in Example 2. The filter medium and the peripheral part of the flexible sheets were integrated by welding in the same manner as in Example 5. The filter medium had a thickness of 9.60 mm, a standard deviation of 0.50 mm, and a thickness at the welded part was 1.40 mm.

[Example 13]

A filter medium was produced in the same manner as in Example 2. The filter medium was put in a 91 mm $\times$ 73.5 mm formwork when welding the filter medium and the peripheral part of the flexible sheets using a high-frequency welding machine to integrate the filter medium and the peripheral part of the flexible sheets by welding. The filter medium had a thickness of 9.60 mm, a standard deviation of 0.70 mm, and a thickness at the welded part of 1.30 mm.

[Example 14]

A blood processing filter was produced in the same manner as in Example 5 except that the filter element had a bulk density of 0.18 (g/m$^3$) and a basis weight of 75.6 (g/m$^2$). The filter medium had a thickness of 9.60 mm, a standard deviation of 0.43 mm, and a thickness at the welded part of 1.45 mm.

[Example 15]

A blood processing filter was produced in the same manner as in Example 2 except that the filter element had a bulk density of 0.28 (g/m$^3$) and a basis weight of 117.6 (g/m$^2$). The filter medium had a thickness of 9.60 mm, a standard deviation of 0.43 mm, and a thickness at the welded part of 1.45 mm.

[Example 16]

A blood processing filter was produced in the same manner as in Example 1 except that the filter element having a thickness of 0.50 (mm) and a basis weight of 105.0 (g/m$^2$) was used and the number of filter elements to be laminated was decreased by one. The filter medium had a thickness of 9.60 mm, a standard deviation of 0.43 mm, and a thickness at the welded part of 1.45 mm.

[Example 17]

A blood processing filter was produced in the same manner as in Example 1. The filter medium had a thickness of 9.60 mm, a standard deviation of 0.35 mm, and a thickness at the welded part of 1.50 mm.

[Example 18]

A blood processing filter was produced in the same manner as in Example 1 except that the filter element having a thickness of 0.38 (mm) and a basis weight of 79.8 (g/m$^2$) was used. The filter medium had a thickness of 7.00 mm, a standard deviation of 0.30 mm, and a thickness at the welded part of 1.45 mm.

[Example 19]

A blood processing filter was produced in the same manner as in Example 2 except that the filter element having a thickness of 0.50 (mm) and a basis weight of 105.0 (g/m$^2$) was used and the number of filter elements to be laminated was decreased by three. The filter medium had a thickness of 12.00 mm, a standard deviation of 0.70 mm, and a thickness at the welded part of 1.40 mm.

[Comparative Example 1]

A blood processing filter was produced in the same manner as in Example 2 except that the welding conditions for the filter medium and the peripheral part of the flexible sheets were changed. The filter medium had a thickness of 9.60 mm, a standard deviation of 0.26 mm, and a thickness at the welded part of 1.85 mm.

[Comparative Example 2]

A blood processing filter was produced in the same manner as in Example 2 except that the welding conditions for the filter medium and the peripheral part of the flexible sheets were changed. The filter medium had a thickness of 9.60 mm, a standard deviation of 0.85 mm, and a thickness at the welded part of 1.20 mm.

[Comparative Example 3]

A blood processing filter was produced in the same manner as in Example 4 except that the same welding conditions as in Comparative Example 1 were employed. The filter medium had a thickness of 9.60 mm, a standard deviation of 0.26 mm, and a thickness at the welded part of 1.85 mm.

[Comparative Example 4]

A blood processing filter was produced in the same manner as in Comparative Example 1 except that the filter element having a bulk density of 0.32 (g/cm$^3$) and a basis weight of 134.4 (g/m$^2$) was used. The filter medium had a thickness of 9.60 mm, a standard deviation of 0.26 mm, and a thickness at the welded part of 1.85 mm.

[Comparative Example 5]

A blood processing filter was produced in the same manner as in Comparative Example 1 except that the same filter medium as in Example 6 was used. The filter medium had a thickness of 9.60 mm, a standard deviation of 0.26 mm, and a thickness at the welded part of 1.85 mm.

[Comparative Example 6]

A blood processing filter was produced in the same manner as in Comparative Example 1 except that the same filter medium as in Example 7 was used. The filter medium had a thickness of 9.60 mm, a standard deviation of 0.26 mm, and a thickness at the welded part of 1.85 mm.

[Comparative Example 7]

A blood processing filter was produced in the same manner as in Comparative Example 1 except that the same filter medium as in Example 8 was used. The filter medium had a thickness of 9.60 mm, a standard deviation of 0.26 mm, and a thickness at the welded part of 1.85 mm.

[Comparative Example 8]

A blood processing filter was produced in the same manner as in Comparative Example 1 except that the same filter medium as in Example 9 was used. The filter medium had a thickness of 9.60 mm, a standard deviation of 0.26 mm, and a thickness at the welded part of 1.85 mm.

[Comparative Example 9]

A blood processing filter was produced in the same manner as in Comparative Example 1 except that the same filter medium as in Example 1 was used. The filter medium had a thickness of 8.00 mm, a standard deviation of 0.18 mm, and a thickness at the welded part of 1.85 mm.

[Comparative Example 10]

A blood processing filter was produced in the same manner as in Example 2 except that two sheets of filter element having a bulk density of 0.21 $(g/cm^3)$ and a basis weight of 88.2 $(g/m^2)$ and 26 sheets of filter element having a bulk density of 0.21 $(g/cm^3)$ and a basis weight of 42.0 $(g/m^2)$ were laminated. The filter medium had a thickness of 9.60 mm, a standard deviation of 0.20 mm, and a thickness at the welded part of 1.45 mm.

The structures and performances of the blood processing filters produced in the above Examples and Comparative Examples are shown in Table 1.

[Table 1]

| | Example 1 | Example 2 | Example 3 | Example 4 | Example 5 | Example 6 | Example 7 | Example 8 | e Example 9 | Example 10 | Example 11 | Example 12 | Example 13 | Example 14 | Example 15 | Example 16 | Example 17 | Example 18 | Example 19 |
|---|---|---|---|---|---|---|---|---|---|---|---|---|---|---|---|---|---|---|---|
| Filter medium thickness (mm) | 8.00 | 9.60 | 11.00 | 9.60 | 9.60 | 9.60 | 9.60 | 9.60 | 9.60 | 8.00 | 8.00 | 9.60 | 9.60 | 9.60 | 9.60 | 9.60 | 9.60 | 7.00 | 12.00 |
| Standard deviation in thickness of filter medium (mm) | 0.30 | 0.43 | 0.80 | 0.43 | 0.43 | 0.43 | 0.43 | 0.43 | 0.43 | 0.30 | 0.35 | 0.50 | 0.70 | 0.43 | 0.43 | 0.43 | 0.35 | 0.30 | 0.70 |
| Bulk density of filter element (g/cm$^3$) | 0.21 | 0.21 | 0.21 | 0.16 | 0.12 | 0.21 | 0.21 | 0.21 | 0.21 | 0.18 | 0.21 | 0.21 | 0.21 | 0.18 | 0.28 | 0.21 | 0.21 | 0.21 | 0.21 |
| Thickness of filter element 1 (mm) | 0.42 | 0.42 | 0.42 | 0.42 | 0.42 | 0.35 | 0.55 | 0.42 | 0.42 | 0.25 | 0.42 | 0.42 | 0.42 | 0.42 | 0.42 | 0.5 | 0.42 | 0.38 | 0.50 |
| Thickness of filter element 2 (mm) | - | - | - | - | - | - | - | - | - | - | - | - | - | - | - | - | - | - | - |

EP 4 115 967 A1

14

| | Example 1 | Example 2 | Example 3 | Example 4 | Example 5 | Example 6 | Example 7 | ExamplE 8 | e Example 9 | Example 10 | Example 11 | Example 12 | Example 13 | Example 14 | Example 15 | Example 16 | Example 17 | Example 18 | Example 19 |
|---|---|---|---|---|---|---|---|---|---|---|---|---|---|---|---|---|---|---|---|
| Effective area (cm²) | 42 | 42 | 42 | 42 | 42 | 42 | 42 | 35 | 55 | 42 | 42 | 42 | 42 | 42 | 42 | 42 | 42 | 42 | 42 |
| Basis weight 1 (g/m²) | 88.2 | 88.2 | 88.2 | 67.2 | 50.4 | 73.5 | 115.5 | 88.2 | 88.2 | 45 | 88.2 | 88.2 | 88.2 | 75.6 | 117.6 | 105 | 88.2 | 79.8 | 105.00 |
| Basis weight 2 (g/m²) | - | - | - | - | - | - | - | - | - | - | - | - | - | - | - | - | - | - | - |
| Thickness at welded part (mm) | 1.45 | 1.45 | 1.30 | 1.45 | 1.45 | 1.45 | 1.45 | 1.45 | 1.45 | 1.50 | 1.45 | 1.40 | 1.30 | 1.45 | 1.45 | 1.45 | 1.50 | 1.45 | 1.40 |
| Filtration time (min) | 28 | 21.1 | 28.0 | 21.1 | 21.1 | 21.1 | 21.1 | 25.3 | 16.1 | 18.0 | 25 | 23 | 24 | 21.5 | 24.8 | 21.1 | 28 | 18 | 29.5 |
| Residual white blood cell count (Loa) | 4.9 | 4.8 | 4.7 | 5.0 | 5.2 | 4.8 | 4.8 | 4.9 | 4.9 | 5.6 | 4.9 | 4.8 | 4.8 | 4.9 | 4.8 | 4.8 | 4.8 | 5.40 | 4.9 |
| Vol loss (mL) | 27.3 | 27.3 | 28 | 28 | 28 | 28.5 | 30 | 21.6 | 38.0 | 20.0 | 27.3 | 27.6 | 27.8 | 27.5 | 27.7 | 27.9 | 27.3 | 20.0 | 29.8 |

EP 4 115 967 A1

| | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|
| Filter medium thickness (mm) | 9.60 | 9.60 | 9.60 | 9.60 | 9.60 | 9.60 | 9.60 | 9.60 | 8.00 | 9.60 |
| Standard deviation in thickness of filter medium (mm) | 0.26 | 0.85 | 0.26 | 0.26 | 0.26 | 0.26 | 0.26 | 0.26 | 0.18 | 0.20 |
| Bulk density of filter element (g/cm$^3$) | 0.21 | 0.21 | 0.16 | 0.32 | 0.21 | 0.21 | 0.21 | 0.21 | 0.21 | 0.21 |
| Thickness of filter element 1 (mm) | 0.42 | 0.42 | 0.42 | 0.42 | 0.35 | 0.55 | 0.42 | 0.42 | 0.42 | 0.42 |
| Thickness of filter element 2 (mm) | - | - | - | - | - | - | - | - | - | 0.20 |

EP 4 115 967 A1

(continued)

| | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|
| Effective area (cm²) | 42 | 42 | 42 | 42 | 42 | 42 | 35 | 55 | 42 | 42 |
| Basis weight1 (g/m²) | 88.2 | 88.2 | 67.2 | 134.4 | 73.5 | 115.5 | 88.2 | 88.2 | 88.2 | 88.2 |
| Basis weight2 (g/m²) | - | - | - | - | - | - | - | - | - | 42.0 |
| Thickness at welded part (mm) | 1.85 | 1.20 | 1.85 | 1.85 | 1.85 | 1.85 | 1.85 | 1.85 | 1.85 | 1.45 |
| Filtration time (min) | 35.0 | 35.0 | 35.0 | 35.0 | 35.0 | 35.0 | 40.0 | 28.0 | 30 | 35 |
| Residual white blood cell count (Loa) | 4.8 | 4.8 | 5.7 | 4.6 | 4.8 | 4.8 | 5.0 | 5.2 | 5.0 | 5.0 |
| Vol loss (mL) | 27.3 | 27.3 | 27.3 | 27.3 | 22.8 | 40 | 21.5 | 38.0 | 27.3 | 27.3 |

Reference Signs List

1 Container, 2 Inlet, 3 Outlet, 4 Filter medium, 5 Space at inlet side, 6 Space at outlet side, 10 Blood processing filter, 11 Filter medium, 12 Welded part

**Claims**

1. A blood processing filter comprising:

   a container having an inlet and an outlet for blood, and
   a filter medium disposed between the inlet and the outlet of the container,
   wherein the filter medium comprises a filter element,
   an average thickness of the filter medium is 7 to 12 mm, and
   a standard deviation in thickness of the filter medium is 0.30 to 0.80 mm.

2. The blood processing filter according to claim 1, wherein the standard deviation in thickness of the filter medium is 0.40 to 0.70 mm.

3. The blood processing filter according to claim 1 or 2,

   wherein the container consists of an inlet-side container material having the inlet and an outlet-side container material having the outlet,
   wherein the inlet-side container material and the outlet-side container material are welded at the periphery thereof while pinching the filter medium, and
   a thickness of the welded part is 1.2 mm to 1.8 mm.

4. The blood processing filter according to any one of claims 1 to 3, wherein the average thickness of the filter medium is 8 to 11 mm.

5. The blood processing filter according to any one of claims 1 to 4, wherein a bulk density of the filter element is 0.14 to 0.30 g/cm$^3$.

6. The blood processing filter according to any one of claims 1 to 5, wherein a bulk density of the filter element is 0.18 to 0.30 g/cm$^3$.

7. The blood processing filter according to any one of claims 1 to 6, wherein an average thickness of the filter element is 0.38 to 0.50 mm.

8. The blood processing filter according to any one of claims 1 to 7, wherein an effective filtration area of the filter medium is 40 to 50 cm$^2$.

[Figure 1]

[Figure 2]

[Figure 3]

[Figure 4]

[Figure 5]

## INTERNATIONAL SEARCH REPORT

| | International application No. |
|---|---|
| | PCT/JP2021/007838 |

**A. CLASSIFICATION OF SUBJECT MATTER**
B01D 39/14(2006.01)i; A61M 1/02(2006.01)i; A61M 1/34(2006.01)i
FI: A61M1/02 107; A61M1/34 100; B01D39/14 Z

According to International Patent Classification (IPC) or to both national classification and IPC

**B. FIELDS SEARCHED**

Minimum documentation searched (classification system followed by classification symbols)
A61M1/02; A61M1/34; B01D39/14

| Documentation searched other than minimum documentation to the extent that such documents are included in the fields searched | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2007-244858 A (ASAHI KASEI MEDICAL CO., LTD.)<br>27 September 2007 (2007-09-27) paragraphs [0016]-<br>[0017], [0031]-[0038], fig. 1-2 | 1-2, 4, 8<br>1-8 |
| Y | JP 2002-291875 A (ASAHI MEDICAL CO., LTD.) 08<br>October 2002 (2002-10-08) paragraphs [0014],<br>[0022] | 1-8 |
| Y | JP 2007-252463 A (ASAHI KASEI MEDICAL CO., LTD.)<br>04 October 2007 (2007-10-04) paragraphs [0057]-<br>[0058] | 3, 5-7 |
| A | JP 4-329965 A (ASAHI MEDICAL CO., LTD.) 18<br>November 1992 (1992-11-18) entire text, all<br>drawings | 1-8 |
| A | JP 2016-112251 A (ASAHI KASEI MEDICAL CO., LTD.)<br>23 June 2016 (2016-06-23) entire text, all<br>drawings | 1-8 |

☐ Further documents are listed in the continuation of Box C.  ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 06 May 2021 (06.05.2021) | 25 May 2021 (25.05.2021) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
|---|---|

Form PCT/ISA/210 (second sheet) (January 2015)

**INTERNATIONAL SEARCH REPORT**

Information on patent family members

International application No.

PCT/JP2021/007838

| Patent Documents referred in the Report | Publication Date | Patent Family | Publication Date |
|---|---|---|---|
| JP 2007-244858 A | 27 Sep. 2007 | US 2007/0199897 A1 paragraphs [0016]-[0029], [0055]-[0062], fig. 1-2 CN 101053683 A | |
| JP 2002-291875 A | 08 Oct. 2002 | (Family: none) | |
| JP 2007-252463 A | 04 Oct. 2007 | (Family: none) | |
| JP 4-329965 A | 18 Nov. 1992 | (Family: none) | |
| JP 2016-112251 A | 23 Jun. 2016 | (Family: none) | |

Form PCT/ISA/210 (patent family annex) (January 2015)

**REFERENCES CITED IN THE DESCRIPTION**

**Patent documents cited in the description**

- JP H2203909 A

- JP 2007050013 A

**Non-patent literature cited in the description**

- Fushokufu no Kiso to Oyo. Japan Textile Machinery Association, 25 August 1993, 119-127

- Guide to the Preparation, Use and Quality Assurance of Blood Components. 2017